(19) Europäisches Patentamt
European Patent Office
Office européen des brevets

(11) Publication number: 0 375 279 A2

# (12) EUROPEAN PATENT APPLICATION

(21) Application number: 89313078.1

(22) Date of filing: 14.12.89

(51) Int. Cl.5: C07C 209/60, C07C 211/10, C07C 211/14

(30) Priority: 21.12.88 JP 320410/88
23.12.88 JP 323469/88
23.12.88 JP 323470/88
23.12.88 JP 323471/88
18.08.89 JP 211341/89

(43) Date of publication of application: 27.06.90 Bulletin 90/26

(84) Designated Contracting States: BE CH DE ES FR GB IT LI NL SE

(71) Applicant: NIPPON SHOKUBAI KAGAKU KOGYO CO. LTD.
1-1, Koraibashi, 4-chome
Chuo-ku Osaka-shi Osaka-fu(JP)

(72) Inventor: Ishikawa, Ryuichi
10-26, Showa-machi Abeno-ku
Osaka-shi Osaka-fu(JP)
Inventor: Tsuneki, Hideaki
5-5-4, Nakanobu
Shinagawa-ku Tokyo(JP)
Inventor: Sugizawa, Hiroshi
27-10-301, Yamadanishi 1-chome
Suita-shi Osaka-fu(JP)
Inventor: Shimasaki, Yuuji
1-305, Nishimachi 5-ban
Takatsuki-shi Osaka-fu(JP)
Inventor: Hayashi, Toshio
2-4-508, Shirakawa 3-chome
Ibaraki-shi Osaka-fu(JP)
Inventor: Hayashi, Tetsuro
5-1-303, Nishimachi
Takatsuki-shi Osaka-fu(JP)
Inventor: Ueshima, Michio
13-63, Hoshoen
Takarazuka-shi Hyogo-ken(JP)

(74) Representative: Myerscough, Philip Boyd et al
J.A.Kemp & Co. 14, South Square Gray's Inn
London, WC1R 5EU(GB)

## (54) Process for producing alkylenamines.

(57) A process for producing an alkylenamine represented by the general formula

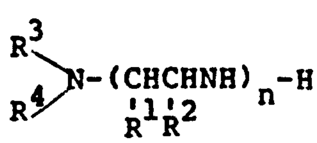

[III]

wherein $R^1$ and $R^2$, independently from each other, represent a hydrogen atom or a methyl or ethyl group, $R^3$ and $R^4$, independently from each other, represent a hydrogen atom, an alkyl group having 1 to 4 carbon atoms, or an aminoalkyl group having 2 to 4 carbon atoms, and n is larger than m and is an integer of 1 to 5,
which comprises reacting an aziridine compound of the general formula

EP 0 375 279 A2

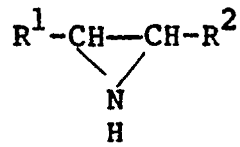

[I]

wherein $R^1$ and $R^2$ are as defined,
with an amine of the general formula

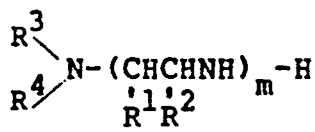

[II]

wherein $R^1$, $R^2$, $R^3$ and $R^4$ are as defined, and m is an integer of 0 to 4,
in the liquid phase in the presence of a solid acid catalyst.

# PROCESS FOR PRODUCING ALKYLENAMINES

This invention relates to a novel process for producing alkylenamines which have extensive utility in various fields such as the textile industry, the rubber industry, agricultural chemicals and medicines.

A known industrial technique of producing alkylenamines involves reacting ethylene dichloride with ammonia or ethylenediamine at high temperatures and pressures to yield ethylenamines such as ethylenediamine, diethylenetriamine and triethylenetetramine (EDC process). This process, however, causes the formation of by-product inorganic salts and vinyl chloride monomer which have to be discharged, and entails extra costs for providing facilities for disposing of these substances or for preventing corrosion by a chlorine ion.

A process involving reacting monoethanolamine with ammonia (MEA process) is also known as another industrial process (Japanese Laid-Open Patent Publication Nos. 236752/1986, 18324/1986 and 94944/1985). By this process, the problems of the EDC process are solved. But it requires high pressures and the amount of by-product cyclic amines is large. Furthermore, a step of separating the desired final product from the by-product amines and the unreacted starting monoethanolamine is complex.

It is also known to synthesize alkylenamines by the reaction of aziridine compounds with ammonia. For example, U. S. Patent No. 2,318,730 discloses the production of ethylenamines by the reaction of ethylenimine with ammonia. In this process, however, the starting ethylenimine consecutively reacts to form polyethylenepolyamines such as diethylenetriamine, triethylenetetramine and tetraethylenepentamine. Hence, this process is unsuitable for the production of only ethylenediamine or another specific alkylenamine as the final desired product. Furthermore, since this process requires the presence of an excessive amount of water in the reaction system, a large amount of energy must be spent for separating the product from the water, and the process is economically disadvantageous.

On the other hand, there are examples in which ethylenimine is reacted with ammonia in a non-aqueous system using a chloride catalyst such as ammonium chloride or aluminum chloride [see Journal of the American Chemical Society, Vol. 70, page 184 (1948), and ibid., Vol. 69, page 2006 (1946)]. However, in this type of process, polyethylenepolyamines form, and since the catalyst forms a salt with the product, it is difficult to separate the product from the catalyst.

It is an object of this invention to solve the problems in the conventional processes for production of alkylenediamines, and to provide a process for producing alkylenediamines highly selectively without involving the formation of by-products such as inorganic salts and vinyl chloride monomer and the corrosion of the equipment as in the EDC process, without requiring high pressures and without involving the formation of by-product cyclic amines as in the MEA process, and furthermore, without involving the problem of separation of the desired alkylenediamines from the reaction product as in the process for synthesizing alkylenediamines by reacting azirididine compounds with ammonia.

Our investigations in an attempt to achieve the foregoing object have now led to the discovery that by reacting aziridine compounds with amines in the liquid phase in the presence of a solid acid catalyst, the reaction proceeds very rapidly under much milder reaction conditions than in the prior art, and alkylenediamines are formed with a very high selectivity.

Thus, according to the present invention, there is provided a process for producing an alkylenamine represented by the general formula

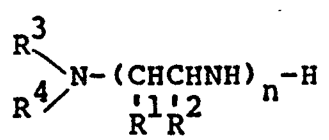

[III]

wherein $R^1$ and $R^2$, independently from each other, represent a hydrogen atom or a methyl or ethyl group, $R^3$ and $R^4$, independently from each other, represent a hydrogen atom, an alkyl group having 1 to 4 carbon atoms, or an aminoalkyl group having 2 to 4 carbon atoms, and n is larger than m and is an integer of 1 to 5,

which comprises reacting an aziridine compound of the general formula

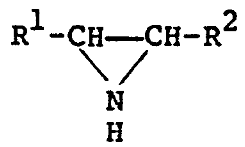

[I]

wherein $R^1$ and $R^2$ are as defined,
with an amine of the general formula

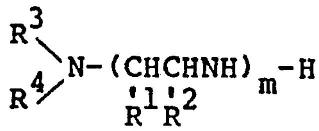

[II]

wherein $R^1$, $R^2$, $R^3$ and $R^4$ are as defined, and m is an integer of 0 to 4,
in the liquid phase in the presence of a solid acid catalyst.

Specific examples of the aziridine compound (I) include ethylenimine, propylenimine and 2-ethylethylenimine. Specific examples of the amine of formula (II) include ammonia; primary amines such as methylamine and ethylamine; secondary amines such as dimethylamine and diethylamine; diamines such as ethylenediamine and methylethylenediamine; and polyamines such as diethylenetriamine and tetraethylenepentamine.

Depending upon the combination of the aziridine compound of formula (I) and the amine of formula (II), the corresponding alkylenamine of formula (III) is obtained. If desired, the compound (I) may be a mixture of the aziridines (I), and the compound (II) may be a mixture of the amines (II). A solvent may be used to lower the reaction pressure.

The solid acid catalyst used in this invention may be any of solid substances having an acidic site on the surface, which may be, for example, (a) cation-exchange resins, (b) solids resulting from bonding of groups containing a protonic acid group to inorganic solids, (c) polyorganosiloxanes containing a protonic acid group, (d) heteropoly acids, (e) isopoly acids, (f) single metal oxides, (g) complex metal oxides, (h) clay minerals, (i) metal sulfates, (j) metal phosphates and (k) metal nitrates. Specific examples are given below.

(a) Cation-exchange resins

Amberlist 15 and Amberlite 200C of Rohm & Haas Co.; Dowex MWC-1-H, Dowex 88 and Dowex HCR-W2 of The Dow Chemical Co.; Lewatit SPC-108 and Lewatit SPC-118 of Bayer AG; Diaion RCP-150H of Mitsubishi Chemical Co., Ltd.; and Sumicaion KC-470, Duolite C-433, and Duolite-464 of Sumitomo Chemical Co., Ltd. and fluorinated sulfonic acid resins (e.g., Nafion-H).

(b) Solids resulting from bonding of groups containing a protonic acid group to inorganic solids

Solids containing a carboxyl group and/or a sulfonic acid group described, for example, in Digiacomo et al., Polyhedron, Vol. 1, page 61 (1982) or Japanese Laid-Open Patent Publication No. 500739/1980, for example, $Zr(O_3PCH_2CH_2SO_3H)_2$, $Zr(O_3PCH_2CH_2CH_2SO_3H)_2$, $Zr(O_3PCH_2CH_2\text{-}(C_6H_4)\text{-}SO_3H)_2$, $Zr(O_3PCH_2CO_2H)_2$, $Zr(O_3PCH_2CH_2CO_2H)_2$, $Zr(O_3PCH_2CH_2CH_2CO_2H)_2$, $Zr(O_3PCH_2CH_2CH_2CH_2CO_2H)_2$, and $Th(O_3PCH_2CH_2CO_2H)_2$.

Solids described in Yoshio Ono et al., NIPPON KAGAKU KAISHI, Vol. 6, page 1111, 1985; specifically, (1) solids obtained by reacting solids having a hydroxyl group on the surface, such as silica, montmorillonite or zirconium phosphate, with a sodium salt of 1,3-propanesultone or diethyl (3-sulfopropyl)phosphonate, and (2) solids having a group containing a sulfonic acid group bonded to the hydroxyl group on the surface, obtained by reacting the above solids with such a compound as trichloro(phenethyl)silane and then sulfonating the benzene ring with chlorosulfuric acid.

(c) Polyorganosiloxanes having a protonic acid group and the organosiloxane having a sulfonic acid group described in Yoshio Ono et al., Journal of Synthetic Organic Chemistry, Japan, Vol. 45, No. 7, page 672 (1987).

(d) Heteropoly acids

Phosphotungstic acid, silicotungstic acid, cobalt-tungstic acid, germanotungstic acid, phosphomolybdic acid, borotungstic acid, and phosphovanadotungstic acid.

(e) Isopoly acids

Niobic acid, tantalic acid, molybdic acid, and tungstic acid

(f) Single metal oxides

Silica, alumina, chromia, zirconia, and titania.

(g) Complex metal oxides

Silica-alumina, silica-magnesia, silica-titania, silica-zirconia, alumina-boria, titania-boria, and zeolites.

(h) Clay minerals

Acid clay, activated clay, montmorillonite, kaolinite and bentonite.

(i) Metal sulfates

Sulfates of metals of Groups I, II, IIIa, IV, V, VIb and VIIIb of the periodic table either as such or supported on carriers. Specific examples of the metals are lithium, sodium, potassium, beryllium, magnesium, chromium, manganese, iron, cobalt, zinc, aluminum, antimony, bismuth, tin and boron.

(j) Metal phosphates

Zirconium phosphate, aluminum phosphate, lanthanum phosphate and boron phosphate, either as such or supported on carriers.

(k) Metal nitrates

Nitrates of metals of Groups I, II, IIIa, IV, V, VIb and VIIIb of the periodic table either as such or supported on carriers. Specific examples of the metals are lithium, sodium, potassium, beryllium, magnesium, chromium, manganese, iron, cobalt, zinc, aluminum, antimony, bismuth, tin and boron.

These solid acids may be used singly or as mixtures in a powdery or granular form. The method of preparing the solid acid catalyst is not particularly limited, and any known method may be used. For example, it may be prepared by calcining a commercial oxide at a temperature of 300 to 800 °C; or by preparing oxides by a customary method from hydroxides or salts or the respective elements, and calcining the oxides.

The reaction temperature is properly prescribed according to the types of the aziridine compound, the amine and the final product, and the catalyst used. Generally, higher reaction temperatures lead to higher reaction pressures, and higher proportions of by-products such as polymers. At lower temperatures, aminoalkylaziridines are formed as by-products. The preferred reaction temperature is 20 to 200 °C.

The reaction pressure may be that under which the reaction materials at the reaction temperature are substantially liquid. It is usually atmospheric pressure to 100 $kg/cm^2$ although it may vary depending upon the types and proportions of the aziridine compound, the amine, and the solvent and the reaction temperature.

A flow-type reactor, a batch-type reactor and a semi-batch-type reactor may be used. From the viewpoint of productivity, a fixed bed flow-type reactor is preferred. In the case of the flow reaction, the space velocity is usually 0.1 to 50 g/gram of the catalyst per hour although it may vary depending upon the catalyst, the reaction temperature, and the reaction materials which are employed in the reaction.

According to this invention, the desired alkylenamines can be obtained efficiently with a reduced amount of by-product cyclic amines. In the present invention, too, some consecutive reactions by which the aziridine compound successively adds to the amino group occur. But as compared with the convention processes, the process of this invention can give alkylenamines with a relatively narrow product distribution. When it is desired to obtain a low-molecular-weight alkylenamine such as diethylenetriamine as a main product, the proportion of the aziridine compound is decreased. When it is desired to obtain a high-molecular-weight alkylenamine such as tetraethylenepentamine as a main product, the proportion of the aziridine compound is increased. If the amount of the aziridine compound is too small, the amount of the alkylenamine is decreased. If, on the other hand, the amount of the aziridine compound is too large, a polymer is formed in a larger amount, and the yield of the desired alkylenamine is decreased disadvantageously. Accordingly, to perform the reaction effectively, the proportions of the starting material charged and the reaction temperatures should preferably be selected appropriately.

The following methods (A) to (F) are especially preferred embodiments of the process of this invention.

(A) A method in which ammonia is used as the amine of formula (II) and reacted with the aziridine compound of formula (I) at a temperature of 20 to 200 °C while adjusting the mole ratio of ammonia to the aziridine of formula (I) to 1-15:1. By this method, the alkylenamine of formula (III) is obtained efficiently with a sharp product distribution.

(B) The same method as (A) except that the reaction temperature is changed to 20 to 100 °C. In this method, the consecutive reactions are further inhibited and an alkylenamine of formula (III) in which n is 1 can be obtained efficiently.

(C) A method in which an amine in which m is 0 (i.e., a primary or secondary amine) is used as the amine of formula (II) and reacted with the aziridine compound of formula (I) at a reaction temperature of 50 to 200 °C while adjusting the mole ratio of the amine to the aziridine compound to 1-15:1. By this method, an alkylenamine of formula (III) having a substituent at the terminal can be efficiently obtained with a sharp product distribution.

(D) A method in which an amine of formula (II) in which m is 1 to 4 is used as the amine of formula (II), and reacted with the aziridine compound of formula (I) at a temperature of 50 to 200 °C while adjusting the mole ratio of the amine to the aziridine compound to 0.1-10:1. By this method, an alkylenamine of formula (III) in which n=m+1 can be obtained efficiently. According to this invention, ethylenediamine is used as the starting material to add 1 mole of ethylenimine, and diethylenetriamine can be obtained as a main product.

(E) A method in which a mixture of ammonia and an amine of formula (II) in which m is 1 is used as the amine of formula (II), and reacted with the aziridine of formula (I) at a temperature of 50 to 200 °C while adjusting the mole ratio of the ammonia/amine/aziridine aziridine compound to 0.1-15/0.05-10/1. By this method, the consecutive reactions are further inhibited, and the product distribution becomes sharp. The desired alkylenamine can be obtained efficiently.

(F) A method in which the aziridine compound of formula (I) is reacted with ammonia in the liquid phase in the presence of the solid acid catalyst (to be referred to as the first-stage reaction), and thereafter adding the aziridine compound of formula (I) further to the reaction mixture containing the resulting amine of formula (II) in which m is 1, and reacting the mixture in the liquid phase in the presence of the solid acid catalyst (to be referred to as the second-stage reaction). By this method, alkylenamines having a sharp product distribution can be efficiently obtained. Particularly, since the reaction product obtained in the first-stage reaction is an alkylenamine, a part of it may be withdrawn and used as the final desired product, as is required.

The following examples illustrate the present invention specifically. It should be understood that the invention should not be limited to them alone.

## EXAMPLE 1

A 300 ml stainless steel autoclave equipped with a magnetic stirrer, a thermometer, a pressure gage and a material feed inlet was charged with 6.14 g of Amberlist 15 (a product of Rohm & Haas Co.), and then the inside of the reactor was purged with nitrogen. Liquid ammonia (68.0 g; 4 moles) was added, and the inside of the reactor was heated to 98 °C. Feeding of ethylenimine was started by using a metering pump. As soon as ethylenimine was fed, a rise in the inside temperature was noted. The inside temperature was controlled to about 100 °C, and over 1 hour, 34.4 g (0.8 mole) of ethylenimine was fed. Thereafter, the reaction was carried out for 2 hours while the inside temperature of the reactor was maintained at 100 °C. After the end of the reaction, the reaction mixture was cooled to room temperature, and the unreacted ammonia was purged slowly.

The reaction mixture was analyzed by an internal standard method using gas-chromatography. The conversion of ethylenimine was 100 %, and the product was found to consist of 16.0 g of ethylenediamine, 14.9 g of diethylenetriamine, 7.3 g of triethylenetetramine, 2.9 parts of tetraethylenepentamine and 0.8 g of a polymer.

## EXAMPLES 2-8

Example 1 was repeated except that the catalysts and reaction conditions shown in Table 1 were used, and the reaction time after the feeding of ethylenimine was changed to 4 hours.

The results are shown in Table 1.

## EXAMPLE 9

One liter of a 1M ethanol solution of aluminum chloride was added to a 2-liter three-necked flask equipped with a stirrer, a reflux condenser and a thermometer, and subsequently, 10 g of montmorillonite (a product of Kunimine Kogyo Co., Ltd.) was added. The mixture was stirred at room temperature for 24 hours. The suspension was then filtered. The solid filtrate was washed with distilled water until no chlorine ion was detected. The solid was then dried in a dryer at 120 °C for 24 hours. As a result, montmorillonite ion-exchanged with an $Al^{3+}$ ion was obtained (catalyst A). The production of catalyst A was in accordance with

the method described by Pierre Laszlo et al., Helvetica Chimica Acta, Vol. 70, page 577 (1987).

Example 1 was repeated except that 8.19 g of catalyst (A) was used instead of Amberlist 15, the conditions shown in Table 1 were used, and the reaction time after the feeding of ethylenimine was changed to 4 hours.

The results are shown in Table 1.

EXAMPLE 10

Into a 250 ml. three-necked flask equipped with a reflux condenser, a stirrer and a thermometer was introduced 21.8 ml of a 38 % aqueous solution of 11.1 g of 2-carboxyethylphosphonic acid in 25 ml of water. Stirring of the solution was started at room temperature, and 9.2 g of $ZrOCl_2$ in 10 ml of water was added. Immediately after the addition, a white precipitate formed. <Water (17 ml) was added to fluidize the solid. By an oil bath, the temperature of the inside of the flask was raised to about 90 to 100 °C, and the mass was refluxed slowly for 15 hours. Then, the resulting slurry was cooled to room temperature, and filtered to separate the solid material. The solid material was then washed with water, acetone and ether, and then dried. Thus, 12.1 g of a catalyst (B) expressed by the empirical formula $Zr(O_3PCH_2CH_2COOH)_2$ was obtained. The production of the catalyst (B) was in accordance with the method of Example 1 of Japanese Laid-Open Patent Publication No. 500739/1980 of P. M. Diogiacomo.

Example 1 was repeated except that 6.14 g of the catalyst (B) was used instead of Amberlist 15 and the reaction time after the feeding of ethylenimine was changed to 4 hours.

The results are shown in Table 1.

EXAMPLE 11

Amberlist 15 (3.6 g) was filled in a stainless steel fixed bed-type continuous reaction apparatus having an inside diameter of 6 mm and a length of 250 mm and equipped with a pressure-adjusting valve and a metering pump, and the inside of the reaction apparatus was filled in ammonia. Then, the apparatus was set in an oil bath heated at 100 °C in such a manner that that side of the apparatus from which the reaction materials were to be introduced faced downwards. A starting mixture composed of ammonia and ethylenimine in a mole ratio of 5 was fed at a rate of 17.5 g/hr under a reaction pressure of 65 $kg/cm^2$.

After 3 hours from the start of feeding the starting mixture, the reaction mixture was analyzed by gas chromatography. The formation of the products indicated in Table 1 was identified.

Table 1

| Example | Reaction conditions | | | | | Conversion of ethylenimine (mole%) | Amount of products yielded (g) | | | | |
|---|---|---|---|---|---|---|---|---|---|---|---|
| | Catalyst | Weight (g) | Temperature (°C) | Reaction materials | | | Ethylenediamine | Diethylenetriamine | Triethylenetetraamine | Tetraethylenepentamine | Polymer |
| | | | | Ethylenimine (mole) | Ammonia/-Ethylenimine (mole ratio) | | | | | | |
| 1 | Amberlist 15 | 6.14 | 100 | 0.8 | 5 | 100 | 16.0 | 14.9 | 7.3 | 2.9 | 0.8 |
| 2 | Silica-alumina | 8.19 | 120 | 0.8 | 5 | 100 | 15.0 | 14.3 | 7.7 | 3.3 | 2.0 |
| 3 | Activated alumina | 8.19 | 120 | 0.8 | 5 | 85 | 12.8 | 12.2 | 6.6 | 2.8 | 1.7 |
| 4 | Bismuth sulfate | 9 24 | 140 | 1.5 | 2 | 100 | 13.9 | 20.3 | 19.0 | 12.7 | 10.2 |
| 5 | Aluminum phosphate | 8.19 | 120 | 0.8 | 5 | 80 | 12.7 | 11.9 | 6.3 | 2.3 | 0.7 |
| 6 | Protonized Y-type zeolite | 8.19 | 120 | 0.8 | 5 | 96 | 13.8 | 13.2 | 7.3 | 3.3 | 2.4 |
| 7 | Silicotungstic acid | 8.19 | 120 | 0.8 | 5 | 100 | 14.5 | 13.9 | 7.7 | 3.5 | 2.5 |
| 8 | Niobic acid | 8.82 | 120 | 0.4 | 10 | 100 | 12.1 | 7.2 | 2.3 | 0.5 | 0.1 |
| 9 | Catalyst (A) | 8.19 | 120 | 0.8 | 5 | 98 | 14.5 | 13.8 | 7.6 | 3.6 | 2.0 |
| 10 | Catalyst (B) | 6.12 | 120 | 0.8 | 10 | 100 | 14.8 | 14.1 | 7.8 | 3.7 | 2.0 |
| 11 | Amberlist 15 | 3.6 | 100 | - | 5 | 100 | 37.6 | 34.8 | 18.6 | 7.0 | 2.1 |

(Note) The amounts of products yielded for Example 11 are composition (% by weight) of the product.

EXAMPLE 12

Four milliliters of trichloro(phenethyl)silane (a product of Shinetsu Chemical Co., Ltd.) and 10 g of silica obtained by evaporating colloidal silica (a product of Nissan Chemical Co., Ltd.) to dryness and calcining the residue at 550 °C for 3 days were refluxed in 100 ml of dioxane for 4 hours. Then, the solid collected by filtration was washed with 30 ml of 1,4-dioxane two times and then with 30 ml of chloroform three times. The resulting silane-modified silica was added to a solution of 40 ml of chlorosulfonic acid in 160 ml of chloroform. The mixture was refluxed for 3 hours and then filtered. The filtrate was washed with 30 ml of chloroform three times, and dried to give a catalyst (C) in which sulfonic acid groups were supported on silica. The production of the catalyst (C) was in accordance with Yoshio Ono et al., NIPPON KAGAKU KAISHI, Vol. 6, page 1111, 1985.

Example 1 was repeated except that 6.14 g of the catalyst (C) was used instead of Amberlist 15, and 45.6 g of propylenimine was used instead of ethylenimine.

The conversion of propylenimine was 100 %. As products, there were obtained 18.8 g of polyamine (1,2-diamino-propane) in which the mole ratio of propylenimine to ammonia was 1, 4.2 g of polyamine in which the above mole ratio was 3, and 2.2 g of a polymer.

EXAMPLE 13

Granular aluminium oxide was crushed to a size of 9 to 16 mesh, and then calcined in air at 500 °C for 2 hours. One gram of this catalyst was packed into a stainless steel reaction tube having an inside diameter of 10 mm, and then the reaction tube was immersed in an oil bath kept at 100 °C. While nitrogen was introduced into the reaction tube, the pressure of the reaction system was adjusted to 40 kg/cm$^2$ by a pressure adjusting valve provided in the outlet of the reaction tube. the nitrogen introducing port was closed, and from the inlet of the reaction tube, a starting mixture composed of ammonia and ethylenimine in a mole ratio of 1:1 was fed at a rate of 50 g/hr.

The reaction products were analyzed by gas chromatography. The results are shown in Table 2.

EXAMPLE 14

Silica-alumina (9-16 mesh) was clacined at 600 °C for 2 hours in air. One gram of the catalyst was used, and a starting mixture composed of ammonia and ethylenimine in a mole ratio of 1:1 was fed at a rate of 10 g/hr and reacted at a temperature of 40 °C under a pressure of 5 kg/cm$^2$ by the same operation as in Example 13.

The results are shown in Table 2.

EXAMPLE 15

Silica-alumina (9-16 mesh) was calcined in air at 600 °C for 2 hours. One gram of the resulting catalyst was used, and a starting mixture composed of ammonia and ethylenimine in a mole ratio of 1:1 was fed at a rate of 5 g/hr and reacted at a temperature of 20 °C under a pressure of 5 kg/cm$^2$ by the same operation as in Example 13.

The results are shown in Table 2.

EXAMPLE 16

Activated clay was kneaded with water. The kneaded mixture was dried in air at 120 °C for 24 hours, calcined in air at 700 °C for 2 hours, and pulverized to a size of 9 to 16 mesh to form a catalyst. One gram of this catalyst was used, and a starting mixture composed of ammonia and ethylenimine in a mole ratio of 5:1 was fed at a rate of 10 g/hr, and reacted at a temperature of 30 °C and a pressure of 30 kg/cm$^2$ by the same operation as in Example 13.

The results are shown in Table 2.

## EXAMPLE 17

Using 1 g of the same catalyst as used in Example 16, a starting mixture composed of ammonia and ethylenimine in a mole ratio of 3:1 was fed at a rate of 20 g/hr, and reacted at a temperature of 80 °C under a pressure of 30 kg/cm$^2$ by the same operation as in Example 13.

The results are shown in Table 2.

## EXAMPLE 18

Using 1 g of the same catalyst as used in Example 16, a starting mixture composed of ammonia and ethylenimine in a mole ratio of 2:1 was fed at a rate of 10 g/hr, and reacted at a temperature of 60 °C under a pressure of 10 kg/cm$^2$ by the same operation as in Example 13.

The results are shown in Table 2.

## EXAMPLE 19

H-mordenite was compression-molded, then calcined in air at 500 °C for 2 hours, and pulverized to a size of 9 to 16 mesh to form a catalyst.

Using 1 g of this catalyst, a starting mixture composed of ammonia and ethylenimine at a mole ratio of 1:1 was fed at a rate of 10 g/hr, and reacted at a temperature of 50 °C under a pressure of 10 kg/cm$^2$ by the same operation as in Example 13.

The results are shown in Table 2.

Table 2

| Example | Catalyst | Ammonia/Ethylenimine (mole ratio) | Feed rate (g/hr) | Temperature (° C) | Pressure (kg/$cm^3$) | Conversion of ethylenimine (mole%) | Composition of the product (wt.%) | | |
|---|---|---|---|---|---|---|---|---|---|
| | | | | | | | Ethylenediamine | Aminoethylaziridine | Polymer |
| 13 | Alumina | 1/1 | 50 | 100 | 40 | 52.8 | 77.7 | 14.8 | 7.5 |
| 14 | Silica-alumina | 1/1 | 10 | 40 | 5 | 38.6 | 82.6 | 17.2 | 0.2 |
| 15 | Silica-alumina | 1/1 | 5 | 20 | 5 | 27.9 | 84.6 | 16.3 | 0.1 |
| 16 | Activeted clay | 5/1 | 10 | 80 | 30 | 44.6 | 85.7 | 13.2 | 1.1 |
| 17 | Activated clay | 3/1 | 20 | 80 | 30 | 38.1 | 88.2 | 11.0 | 0.8 |
| 18 | Activated clay | 2/1 | 10 | 60 | 10 | 34.1 | 83.9 | 15.4 | 0.7 |
| 19 | H-mordenite | 1/1 | 10 | 50 | 10 | 36.2 | 87.2 | 10.8 | 2.0 |

EXAMPLE 20

Kaolin was kneaded with water. The kneaded mixture was dried in air at 120 °C for 24 hours, calcined at 400 °C for 2 hours, and pulverized to a size of 9 to 16 mesh to form a catalyst. Using 1 g of the resulting catalyst, a starting mixture composed of ammonia and propylenimine in a mole ratio of 1:1 was fed at a rate of 10 g/hr, and reacted at a temperature of 60 °C under a pressure of 10 kg/$cm^2$ by the same operation as in Example 13.

The results are shown in Table 3.

Table 3

| | | |
|---|---|---|
| Conversion of propylenimine (mole%) | | 41.9 |
| Composition of the product (wt.%) | 1,2-diamino-propane | 92.0 |
| | 1-Amino-2-methyl-ethyl-propylenimine | 7.8 |
| | Dimethylethylenetriamine | 0.2 |

EXAMPLE 21

Bentonite was kneaded with water. The kneaded mixture was dried in air at 120 °C for 24 hours, calcined at 300 °C for 2 hours, and pulverized to a size of 9 to 16 mesh to form a catalyst. Using 1 g of this catalyst, a starting material composed of ammonia and 2-ethyl-ethylenimine in a mole ratio of 2:1 was fed at a rate of 10 g/hr, and reacted at a temperature of 60 °C under a pressure of 10 kg/$cm^2$ by the same operation as in Example 13.

The results are shown in Table 4.

Table 4

| | | |
|---|---|---|
| Conversion of 2-ethylethylenimine (mole%) | | 41.9 |
| Composition of the product (wt.%) | 1,2-diamino-butane | 87.7 |
| | N-(1-aminomethylpropane)-2-ethylethylenimine | 9.8 |
| | Dimethylethylenetriamine | 2.5 |

EXAMPLE 22

Bismuth sulfate (7.4 g) and 72.1 g of ethylenediamine were introduced into a 300 ml stainless steel autoclave equipped with a magnetic stirrer, a thermometer, a pressure gage and a material feed inlet, and then the inside of the reactor was replaced by nitrogen. By using an oil bath, the temperature of the inside

of the reactor was elevated to 118 ° C. Ethylenimine (51.6 g) was fed from the feed inlet over one hour using a metering pump. With the start of feeding ethylenimine, a rise in the inside temperature was noted. The inside temperature of the reactor was controlled and maintained at about 120 ° C. After a predetermined amount of ethylenimine was fed, the reaction was further continued for 4 hours while the temperature was maintained at 120 ° C. After the reaction, the reaction mixture was cooled to room temperature and analyzed by using gas chromatography in accordance with an internal standard method.

The conversion of ethylenimine was 100 %, and the conversion of ethylenediamine was 68 %. The product consisted of 53.1 g of diethylenetriamine, 35.2 g of triethylenetetramine, 8.6 g of tetraethylenepentamine and 3.8 g of a polymer.

## EXAMPLE 23

Example 22 was repeated except that 10.6 g of iron sulfate was used instead of bismuth sulfate, the reaction temperature was changed to 140 ° C, and 104.1 g of ethylenimine was fed.

The results are shown in Table 5.

## EXAMPLE 24

Four grams of zinc sulfate and 72.0 g of ethylenediamine were introduced into the same reactor as used in Example 22, and the inside of the reactor was purged by nitrogen. After the temperature of the inside of the reactor was elevated to l08 ° C, a mixture of 25.8 g of etylenimine and 36 g of ethylenediamine was fed from the material feed inlet over 1 hour by means of a metering pump. Since a rise in the inside temperature was noted with the start of feeding the starting mixture, the reaction temperature was controlled, and maintained at about 110 ° C. After the end of feeding the starting mixture, the reaction was further continued for 4 hours at 110 ° C, and thereafter the same operation as in Example 22 was performed.

The results are shown in Table 5.

## EXAMPLE 25

Example 22 was repeated except that 10 g of ammonium sulfate was used instead of bismuth sulfate, and the reaction temperature was changed to 100 ° C.

The results are shown in Table 5.

## EXAMPLE 26

Example 22 was repeated except that 7.4 g of ammonium nitrate was used instead of bismuth sulfate, and the reaction temperature was changed to 110 ° C.

The results are are shown in Table 5.

Table 5

| Example | Catalyst | | Reaction materials | | Temperature (°C) | Conversion of ethylenimine (%) | Conversion of ethylenediamine (%) | Amounts of the products (g) | | | |
|---|---|---|---|---|---|---|---|---|---|---|---|
| | Type | weight (g) | Ethylenimine (mole) | Mole ratio of ethylenediamine to ethylenimine | | | | Diethylenetriamine | Triethylenetetramine | Tetraethylenepentamine | Polymer |
| 22 | Bismuth sulfate | 7.4 | 1.2 | 1.0 | 120 | 100 | 68 | 53.1 | 35.2 | 8.6 | 3.8 |
| 23 | Iron sulfate | 10.6 | 2.4 | 0.5 | 140 | 100 | 93.8 | 33.2 | 65.0 | 50.3 | 23.2 |
| 24 | Zinc sulfate | 4 | 0.4 | 3 | 110 | 100 | 28.2 | 43.2 | 12.1 | - | - |
| 25 | Ammonium sulfate | 10 | 1.2 | 1 | 100 | 96 | 67.1 | 53.1 | 34.7 | 8.6 | 1.5 |
| 26 | Ammonium nitrate | 7.4 | 1.2 | 1 | 110 | 100 | 67 | 51.3 | 33.0 | 13.2 | 2.4 |

## EXAMPLE 27

Bismuth sulfate (3.5 g) was filled into a stainless steel fixed bed-type continuous reaction apparatus having an inside diameter of 6 mm and a thickness of 250 mm and equipped with a pressure adjusting valve and a metering pump. The reaction system was filled with ethylenediamine, and the reaction apparatus was set in an oil bath kept at 120 °C in such a manner that the side of the oil bath from which the reaction materials were to be fed faced downwards. A starting mixture composed of ethylenimine and ethylenediamine in a mole ratio of 1:3 was fed into the reaction apparatus at a rate of 14 g/hr under a reaction pressure of 2.5 kg/cm$^2$.

After 3 hours from the start of feeding the starting mixture, the data shown in Table 6 were obtained.

Table 6

| | | |
|---|---|---|
| Conversion of ethylenimine (%) | | 100 |
| Conversion of ethylenediamine (%) | | 29.8 |
| Composition of the product (wt.%) | Ethylenediamine | 56.8 |
| | Diethylenetriamine | 36.1 |
| | Triethylenetetramine | 6.5 |
| | Tetraethylenepentamine | 0.5 |
| | Polymer | 0.2 |

## EXAMPLE 28

Example 22 was repeated except that 10 g of beryllium sulfate was used instead of bismuth sulfate, and 68.4 g of propylenimine was used instead of ethylenimine.

The conversion of propylenimine was 100 %, and the conversion of ethylenediamine was 69 %. The product consisted of 61.1 g of polyamine in which the mole ratio of propylenimine to ethylenediamine was 1, 43.0 g of a polyamine in which this mole ratio was 2, 12.2 g of a polyamine in which the mole ratio was 3, and 1.9 g of polymer.

## EXAMPLE 29

A 300 ml stainless steel autoclave equipped with a magnetic stirrer, a thermometer, a pressure gage and a material feed inlet was charged with 3.7 g of Amberlist 15 and 72.1 g of ethylenediamine, and then the inside of the reactor was purged with nitrogen. The temperature of the inside of the reactor was elevated to 118 °C by an oil bath. Then, 51.6 g of ethylenimine was fed from the feed inlet over 1 hour by means of a metering pump. Since a rise in the inside temperature was noted with the start of feeding ethylenimine, the inside temperature of the reactor was controlled and maintained at about 120 °C. After a predetermined amount of ethylenimine was fed, the reaction was continued for 2 hours while the temperature was maintained at 120 °C. After the reaction, the reaction mixture was cooled to room temperature, and the catalyst was separated by filtration.

The reaction mixture was analyzed by gas chromatography by an internal standard method. The conversion of ethylenimine was 100 %, and the conversion of ethylenediamine was 69 %. The product consisted of 53.8 g of diethylenetriamine, 36.1 g of triethylenetetramine, 10.0 g of tetraethylenepentamine

and 1.5 g of a polymer.

## EXAMPLE 30

Example 29 was repeated except that instead of Amberlist 15, 7.4 g of ethylenediamine salt of Dowex MWC-1-H (a product of Dow Chemical Co.), was used and the reaction temperature was changed to 100 °C. The results are shown in Table 7.

## EXAMPLE 31

The catalyst (B) prepared in Example 10 (4.0 g) and 72.0 g of ethylenediamine were fed into the same reactor as used in Example 29, and the inside of the reactor was purged with nitrogen. The inside temperature was raised to 98 °C. Then, a mixture of 25.8 g of ethylenimine and 36 g of ethylenediamine was fed into the reactor from the material feed inlet over 1 hour by means of a metering pump. Since a rise in the inside temperature was noted with the start of feeding the starting mixture, the reaction temperature was controlled and maintained at about 100 °C. After the end of feeding the starting mixture, the reaction was continued for 3 hours at 100 °C. Thereafter, the same operation as in Example 29 was performed.

The results are shown in Table 7.

## EXAMPLE 32

Example 29 was repeated except that 10 g of the catalyst (C) prepared in Example 12 was used instead of Amberlist 15, and the reaction temperature was changed to 100 °C.

The results are shown in Table 7.

## EXAMPLE 33

Ethanol (125 ml) was added to a 500 ml four-necked flask equipped with a stirrer, a dropping funnel, a thermometer and a distillation device, and 145.6 g (0.7 mole) of tetraethoxysilane and 72. 1 g (0.3 mole) of phenyltriethoxysilane were dissolved. With stirring, 35 ml of a 0.01M HCl solution was added dropwise to this solution over 1 hour. Thereafter, the flask was heated over an oil bath heated at 125 °C, and ethanol was distilled out until the volume of the reaction mixture became 120 ml. The reaction mixture was cooled to 15 °C, and 60 ml of ethanol and 90 ml of cyclohexane were added. With vigorous stirring, 270 ml of water and 50 ml of 28 % aqueous ammonia were added, and the mixture was stirred for 4 hours. The resulting precipitate was separated by filtration, washed with distilled water, and dried in vacuum at 120 °C for 4 hours to give polysiloxane containing phenyl groups. Then, 4.37 g of chlorosulfonic acid and 18 ml of chloroform were put in a 100 ml four-necked flask equipped with a stirrer, a thermometer and a reflux condenser. Subsequently, 10 g of the polysiloxane was added, and the mixture was refluxed for 3 hours to perform sulfonation and thus to give sulfonated polyorganosiloxane (catalyst D) of the following formula.

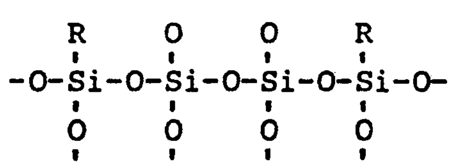

$(R = C_6H_4SO_3H)$

Example 29 was repeated except that 5.3 g of the catalyst D was used instead of Amberlist 15, the reaction temperature was changed to 80 °C, and 104.1 g of ethylenimine was fed.

The results are shown in Table 7.

## EXAMPLE 34

Example 29 was repeated except that 6.2 g of silicotungstic acid was used instead of Amberlist 15.

The results are shown in Table 7.

## EXAMPLE 35

Example 29 was repeated except that 6.2 g of niobic acid was used instead of Amberlist 15.

The results are shown in Table 7.

Table 7

| Example | Catalyst | | Reaction materials | | Temperature (°C) | Conversion of ethylenimine (%) | Conversion of ethylenediamine (%) | Amounts of the products (g) | | | |
|---|---|---|---|---|---|---|---|---|---|---|---|
| | Type | weight (g) | Ethylenimine (mole) | Mole ratio of ethylenediamine to ethylenimine | | | | Diethylenetriamine | Triethylenetetramine | Tetraethylenepentamine | Polymer |
| 29 | Amberlist 15 | 3.7 | 1.2 | 1.0 | 120 | 100 | 69 | 53.8 | 36.1 | 10.1 | 1.5 |
| 30 | Dowex MWC-1-H ethylenediamine salt | 7.4 | 1.2 | 1.0 | 100 | 100 | 68 | 33.1 | 35.2 | 8.6 | 3.8 |
| 31 | Catalyst (B) | 4.0 | 0.6 | 3.0 | 100 | 100 | 28.2 | 43.2 | 12.1 | 0.9 | 0.1 |
| 32 | Catalyst (C) | 10 | 1.2 | 1 | 100 | 96 | 67.1 | 53.1 | 34.7 | 8.6 | 1.5 |
| 33 | Catalyst (D) | 5.3 | 2.4 | 0.5 | 80 | 100 | 93.8 | 33.2 | 65.0 | 50.3 | 23.2 |

- to be continued -

Table 7 (continued)

| Example | Catalyst | | Reaction materials | | Temperature (°C) | Conversion of ethylenimine (%) | Conversion of ethylenediamine (%) | Amounts of the products (g) | | | |
|---|---|---|---|---|---|---|---|---|---|---|---|
| | Type | weight (g) | Ethylenimine (mole) | Mole ratio of ethylenediamine to ethylenimine | | | | Diethylenetriamine | Triethylenetetramine | Tetraethylenepentamine | Polymer |
| 34 | Silicotungstic acid | 6.2 | 1.2 | 1.0 | 120 | 100 | 66.8 | 50.1 | 35.6 | 11.1 | 3.0 |
| 35 | Niobic acid | 6.2 | 1.2 | 1.0 | 120 | 100 | 67 | 51.3 | 33.0 | 13.2 | 2.4 |

## EXAMPLE 36

Amberlist 15 (3.5 g) was filled in a stainless steel fixed bed continuous reaction apparatus having an inside diameter of 6 mm and a length of 250 mm and equipped with a pressure adjusting valve and a metering pump, and the reaction system was filled with ethylenediamine. The apparatus was then set in an oil bath kept at 120 °C in such a manner that that side of the apparatus from which the reaction materials were to be fed faced downwards. A starting mixture composed of ethylenimine and ethylenediamine in a mole ratio of 1:3 was fed under a reaction pressure of 2.5 kg/cm$^2$ at a rate of 52.5 g/hr. Three hours after the start of feeding the materials, the reaction mixture was analyzed by gas chromatography.

The results are shown in Table 8.

Table 8

| | | |
|---|---|---|
| Conversion of ethylenimine (%) | | 94.8 |
| Conversion of ethylenediamine (%) | | 27.3 |
| Composition of the product (wt.%) | Ethylenediamine | 58.7 |
| | Diethylenetriamine | 32.0 |
| | Triethylenetetramine | 8.1 |
| | Polymer | 0.2 |

## EXAMPLE 37

Example 36 was repeated except that 3.5 g of niobic acid was used instead of Amberlist 15, the reaction temperature waschanged to 90 °C, and a starting mixture composed of ethylenimine and ethylenediamine in a mole ratio of 2:1 was fed under a reaction pressure of 1.5 kg/cm$^2$ at a rate of 14 g/hr.

After 3 hours from the start of feeding the materials, the data shown in Table 9 were obained.

Table 9

| | | |
|---|---|---|
| Conversion of ethylenimine (%) | | 100 |
| Conversion of ethylenediamine (%) | | 93.9 |
| Composition of the product (wt.%) | Ethylenediamine | 2.6 |
| | Diethylenetriamine | 18.8 |
| | Triethylenetetramine | 36.9 |
| | Tetraethylenepentamine | 28.5 |
| | Polymer | 13.2 |

## EXAMPLE 38

Example 29 was repeated except that 68.4 g of propylenimine was used instead of ethylenimine.

The conversion of propylenimine was 100 %, and the conversion of ethylenediamine was 69 %. The product consisted of 61.1 g of a polyamine in which the mole ratio of propylenimine to ethylenediamine was 1, 43.0 g of a polyamine in which the mole ratio was 2, 12.2 g of a polyamine in which the mole ratio was 3, and 1.9 g of a polymer.

## EXAMPLE 39

Amberlist 15 (4.62 g) was introduced into a 300 ml stainless steel autoclave equipped with a magnetic stirrer, a thermometer, a pressure gage and a material feed inlet, and the inside of the reactor was purged with nitrogen. Liquid ammonia (34 g; 2 moles) was added, and the temperaure of the inside of the reactor was elevated to 98 °C by an oil bath. A mixture of 43 g (1 mole) of ethylenimine and 15 g (0.25 mole) of ethylenediamine was fed by a metering pump. Since a rise in the inside temperature was noted with the start of feeding the starting mixture, feeding of the starting mixture was continued while the inside temperature of the reactor was maintained at about 100 °C. Then, the reaction was continued for 2 hours while the temperature of the inside of the reactor was maintained at 100 °C. After the end of the reaction, the reaction mixture was cooled to room temperature, and the unreacted ammonia was slowly discharged.

The reaction mixture was analyzed by gas chromatography in accordance with an internal standard method. The conversion of ethylenimine was 100 %. The product consisted of 1.59 g (the value excepting the weight percent of the ethylenediamine fed), 20.29 g of diethylenetriamine, 15.56 g of triethylenetetramine, 8.17 g of tetraethylenepentamine, and 4.21 g of a polymer.

The reaction conditions and the reaction results are shown in Table 10.

## EXAMPLE 40

Example 39 was repeated except that 8.19 g of silica-alumina was used instead of Amberlist 15, 68 g of ammonia was used, 24 g of ethylenediamine was used, and the reaction temperature was changed to 120 °C.

The results are shown in Table 10.

## EXAMPLE 41

Example 39 was repeated except that 4.28 g of niobic acid was used instead of Amberlist 15, 68 g of ammonia was used, 24 g of ethylenediamine was used, and the reaction temperature was changed to 120 °C.

The results are shown in Table 10.

## EXAMPLES 42-46

Example 39 was repeated except that the catalyst and th reaction conditions were changed as shown in Table 10.

The results are shown in Table 10.

## EXAMPLE 47

Example 39 was repeated except that the catalyst (A) prepared in Example 9 was used instead of

Amberlist 15, and the reaction conditions were changed as indicated in Table 10.
The results are shown in Table 10.

## EXAMPLES 48

Example 39 was repeated except that instead of Amberlist 15, the catalyst (B) prepared in Example 10 was used instead of Amberlist 15.
The results are shown in Table 10.

## EXAMPLE 49

Example 39 was repeated except that instead of Amberlist 15, the catalyst (C) prepared in Example 12 was used.
The results are shown in Table 10.

## EXAMPLE 50

Amberlist 15 (3.5 g) was filled in a stainless steel fixed bed-type continuous reaction apparatus having an inside diameter of 6 mm and a length of 250 mm and equipped with a pressure adjusting valve and a metering pump, and the reaction system was filled with ammonia. The reaction apparatus was set in an oil bath kept at 100 °C in such a manner that that side of the reaction apparatus from which the materials were to be fed faced downwards. A starting mixture composed of ammonia, ethylenimine and ethylenediamine in a mole ratio of 3:1:0.25 was fed at a rate of 17.5 g/hr under a reaction pressure of 65 $kg/cm^2$.
After 3 hours from the start of feeding the starting mixture, the reaction mixture was analyzed by gas chromatography, and the results obtained are shown in Table 10.

Table 10

| Example | Catalyst | | Temperature (°C) | Reaction materials | | | Conversion of ethylenimine (%) |
|---|---|---|---|---|---|---|---|
| | Type | weight (g) | | Ethylenimine (mole) | Mole ratio of ammonia to ethylenimine | Mole ratio of ethylenediamine to ethylenimine | |
| 39 | Amberlist 15 | 4.62 | 100 | 1 | 2 | 0.25 | 100 |
| 40 | Silica-alumina | 8.10 | 120 | 1 | 4 | 0.4 | 100 |
| 41 | Niobic acid | 4.26 | 120 | 1 | 2 | 0.25 | 100 |
| 42 | Bismuth sulfate | 7.15 | 140 | 0.3 | 15 | 1 | 100 |
| 43 | Silicotungstic acid | 4.47 | 120 | 0.3 | 15 | 3 | 100 |
| 44 | Protonized Y-type zeolite | 8.10 | 120 | 0.8 | 5 | 0.25 | 100 |
| 45 | Aluminum phosphate | 8.10 | 120 | 0.8 | 5 | 1 | 88 |
| 46 | Activated $\gamma$-alumina | 8.10 | 120 | 0.8 | 5 | 0.5 | 91 |
| 47 | Catalyst (A) | 7.52 | 120 | 1.0 | 3 | 0.1 | 100 |
| 48 | Catalyst (B) | 7.52 | 100 | 1.0 | 3 | 0.25 | 100 |
| 49 | Catalyst (C) | 7.52 | 100 | 1.0 | 3 | 0.5 | 100 |
| 50 | Amberlist 15 | 3.5 | 100 | | 3 | 0.25 | 96 |

- to be continued -

Table 10 (continued)

| Example | Composition of the product (wt.%) (*) Ethylenediamine | Diethylenetriamine | Triethylenetetramine | Tetraethylenepentamine | Polymer | Amount of the products yielded (g) |
|---|---|---|---|---|---|---|
| 39 | 3.2 | 40.7 | 31.2 | 16.4 | 8.5 | 49.8 |
| 40 | 17.8 | 53.3 | 21.8 | 5.8 | 1.3 | 41.5 |
| 41 | 25.5 | 59.1 | 13.8 | 1.4 | 0.2 | 21.2 |
| 42 | 27.7 | 54.7 | 15.2 | 2.2 | 0.2 | 27.1 |
| 43 | 31.3 | 60.3 | 7.8 | 0.6 | 0.0 | 16.1 |
| 44 | 26.6 | 45.1 | 20.7 | 6.1 | 1.5 | 42.0 |
| 45 | 0.3 | 69.7 | 23.8 | 5.4 | 0.8 | 35.7 |
| 46 | 17.8 | 53.3 | 21.8 | 5.8 | 1.3 | 59.5 |
| 47 | 21.2 | 38.5 | 26.2 | 11.8 | 2.3 | 51.6 |
| 48 | 11.7 | 48.7 | 28.6 | 9.3 | 1.7 | 51.0 |
| 49 | 2.1 | 54.6 | 29.4 | 10.6 | 3.3 | 50.1 |
| 50 | 11.7 | 48.7 | 28.6 | 9.3 | 1.7 | |

(*): The unreacted ammonia, the ethylendiamine fed, and the unreacted ethylenimine were removed from the product.

## EXAMPLE 51

A 500 ml stainless steel autoclave equipped with a magnetic stirrer, a thermometer, a pressure gage and a material feed inlet was charged with 3.9 g of Dowex-HCR W2 (H-type) as a catalyst, and the inside of the reactor was purged with nitrogen. Dimethylamine (135 g; 3 moles) was introduced into the autoclave, and the temperature was raised to 110 °C. At this temperature, 43 g (1 mole) of ethylenimine was added with stirring, and after the end of the addition, the mixture was further stirred at 110 °C for 2 hours. The reaction during this time was carried out under autogenous pressure.

The reaction product was cooled, taken out, and analyzed by gas chromatography. It was determined that the reaction product consisted of 74.8 g of N,N-dimethyl-ethylenediamine, 6.6 g of N,N-dimethyl-N′-aminoethyl-ethylenediamine and 2.8 g of other polyamines.

## EXAMPLE 52

Example 51 was repeated except that the catalyst (A) prepared in Example 9 was used instead of the catalyst used in Example 51, and the reaction conditions were changed as shown in Table 11.

The results are shown in Table 11.

## EXAMPLES 53-55

Example 51 was repeated except that the reaction conditions were changed as indicated in Table 11.

The results are shown in Table 11.

Table 11

| Example | Reaction material | | | | | | | Results | |
|---|---|---|---|---|---|---|---|---|---|
| | Catalyst | | Starting material | | | Conditions | | Conversion of the aziridine (%) | Amounts of the products yielded (g) |
| | Type | Amount (g) | Amine (g) | Aziridine (g) | Amine to aziridine (mole ratio) | Temperature (°C) | Time (hr) | | |
| 51 | Dowex HCR-W2 | 3.9 | dimethylamine (135) | ethylenimine (43) | 3 | 110 | 4 | 100 | N,N-dimethylethylenediamine (74.8)<br>N,N-dimethyl-N'-amino-ethylethylenediamine (6.6)<br>Other polyamines (2.8) |
| 52 | Catalyst (A) | 4.0 | diethylamine (292) | ethylenimine (34.4) | 5 | 120 | 4 | 100 | N,N-diethylethylenediamine (76.1)<br>N,N-diethyl-N'-aminoethylethylenediamine (8.3)<br>Other polyamines (2.5) |
| 53 | Silicotungstic acid | 4.0 | diethylamine (365) | propylenimine (28.5) | 10 | 90 | 4 | 98 | N,N-diethylpropylenediamine (51.7)<br>Other polyamines (2.7) |

- to be continued -

Table 11 (continued)

| Example | Reaction material | | | | | | | Results | |
|---|---|---|---|---|---|---|---|---|---|
| | Catalyst | | Starting material | | | Conditions | | Conversion of the aziridine (%) | Amounts of the products yielded (g) |
| | Type | Amount (g) | Amine (g) | Aziridine (g) | Amine to aziridine (mole ratio) | Temperature (°C) | Time (hr) | | |
| 54 | Niobic acid | 4.0 | ethylamine (135) | ethylenimine (43) | 3 | 100 | 4 | 100 | N-ethylethylendiamine (73.1)<br>Other polyamines (9.2) |
| 55 | Activated clay | 4.1 | dimethylamine (90) | ethylenimine (86) | 1 | 120 | 4 | 100 | N,N-dimethylethylenediamine (43.1)<br>N,N-dimethyl-N'-aminoethylethylenediamine (25.9)<br>Other polyamines (6.6) |

The process of this invention does not use compounds having chlorine or a hydroxyl group as starting materials. Accordingly, no chlorine-containing materials result which are attributed to the starting materials and which have to be discarded. This offers the advantage that since no by-product having a hydroxyl group which is difficult to separate from the alkylenamines forms, the resulting products are highly pure. In addition, since the reaction does not have to be performed in the presence of water, no step is necessary for separating water.

Moreover, since in the present invention, solid acids which do not dissolve in, nor are dissolved by, the reaction products are used, the resulting alkylenamines can be very easily separated from the catalyst. Another advantage is that since the solid acid catalyst permits the reaction of the aziridine compound and the amine to proceed very rapidly, the reaction can be carried out under unprecedented mild reaction conditions.

## Claims

1. A process for producing an alkylenamine of the general formula

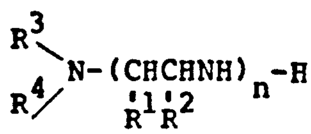

[III]

wherein $R^1$ and $R^2$, independently from each other, represent a hydrogen atom or a methyl or ethyl group, $R^3$ and $R^4$, independently from each other, represent a hydrogen atom, an alkyl group having 1 to 4 carbon atoms or an aminoalkyl group having 2 to 4 carbon atoms, and n is an integer of 1 to 5, which comprises reacting an aziridine compound of the general formula

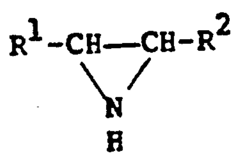

[I]

wherein $R^1$ and $R^2$ are as defined, with an amine of the general formula

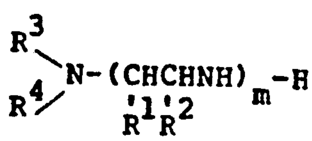

[II]

wherein $R^1$, $R^2$, $R^3$, $R^4$ are as defined, and m is smaller than n and is an integer of 0 to 4, in the liquid phase in the presence of a solid acid catalyst.

2. A process according to claim 1 in which the amine of formula (II) is ammonia.

3. A process according to claim 2 in which the mole ratio of ammonia to the aziridine compound of formula (I) is 1-15:1, and the reaction is carried out at a temperature of 20 to 200°C.

4. A process according to claim 3 in which the reaction is carried out at a temperature of 20 to 100°C, and an alkylenamine of formula (III) in which n is 1 is obtained as a main product.

5. A process according to any one of claims 2 to 4 in which an aziridine compound of formula (I) is first reacted with ammonia to provide a reaction mixture containing an amine of formula (II) wherein m is 1, and is then reacted with the reaction mixture to provide an alkylenamine of formula (III) wherein n is at least 2.

6. A process according to claim 1 in which the amine of formula (II) is one in which m is 0, the mole ratio of the amine of formula (II) to the aziridine compound of formula (I) is 1-15:1, and the reaction is carried out at a temperature of 50 to 200°C.

7. A process according to claim 1 in which the amine of formula (II) is one in which m is 1 to 4, the mole ratio of the amine of formula (II) to the aziridine compound is 0.1-10:1, the reaction is carried out at a

temperature of 50 to 200 °C, and an alkylenamine of formula (III) in which n = m + 1 is obtained as a main product.

8. A process according to claim 1 in which the amine of formula (II) is a mixture of ammonia and an amine of formula (II) in which m is 1, the mole ration of ammonia/the amine of formula (II) in which m is 1/the aziridine compound of formula (I) is 0.1-15:0.05-10:1, and the reaction is carried out at a temperature of 50 to 200 °C.

9. A process according to any one of claims 1 to 8 in which the solid acid catalyst is at least one of (a) a cation-exchange resin, (b) a solid resulting from bonding a group containing a protonic acid group to an inorganic solid, (c) a polyorganosiloxane containing a protonic acid group, (d) a heteropoly acid, (e) an isopoly acid, (f) a simple metal oxide, (g) a complex metal oxide, (h) a clay mineral, (i) a metal sulfate, (j) a metal phosphate and (k) a metal nitrate.

10. A process according to any one of claims 1 to 9 in which the aziridine compound of formula (I) is ethylenimine.

11. A process according to any one of claims 1 and 7 to 10 in which the amine of formula (II) is ethylenediamine.